# EUROPEAN PATENT APPLICATION

(11) **EP 3 865 133 A1**
(43) Date of publication of application: **18.08.2021**
(21) Application number: 19871138.4
(22) Date of filing: 11.10.2019
(51) Int. Cl.: A61K 31/505, A61P 35/02, A61P 43/00

(54) **ANTITUMOR AGENT FOR ACUTE MYELOID LEUKEMIA**

(30) Priority: 12.10.2018 JP 2018193098; 08.02.2019 JP 2019021165
(71) Applicant: FUJIFILM Corporation, Tokyo 106-8620 (JP)
(72) Inventor: ANDO Makoto, Ashigarakami-gun, Kanagawa 258-8577 (JP); WATABE Satoshi, Tokyo 106-0031 (JP)
(74) Representative: Hoffmann Eitle
(86) International application number: PCT/JP2019/040190
(87) International publication number: WO 2020/075838

(57) **Abstract**

There is provided an antitumor agent for acute myeloid leukemia, which exhibits a practical curing effect on acute myeloid leukemia. According to the present invention, there is provided the antitumor agent for acute myeloid leukemia, whose dose per administration is within a predetermined range and which contains a compound represented by General Formula [1] specified in the present specification, such as (S,E)-N-(1-((5-(2-((4-cyanophenyl)amino)-4-(propylamino)pyrimidin-5-yl)-4-pentyn-1-yl)ami no)-1-oxopropan-2-yl)-4-(dimethylamino)-N-methyl-2-butenamide or a salt thereof.

## Description

### Field of the Invention

The present invention relates to an antitumor agent for acute myeloid leukemia.

### Description of the Related Art

A nitrogen-containing heterocyclic compound having an excellent Fms-like tyrosine kinase 3 (FLT3) inhibitory activity and being useful as a drug substance for pharmaceuticals has been reported (Patent Documents 1 and 2). In addition, a pharmaceutical composition for treating FLT3 mutation-positive cancer, which contains the above nitrogen-containing heterocyclic compound, has been reported (Patent Document 3). Further, a method for producing the nitrogen-containing heterocyclic compound and an intermediate thereof have been reported (Patent Document 4). Hereinafter, the compound represented by General Formula [1] described in Patent Document 3 or a salt thereof may be simply referred to as Compound A.

### Prior Art Documents

### Patent Documents

Patent Document 1: WO2013/157540A
Patent Document 2: WO2015/056683A
Patent Document 3: WO2016/027904A
Patent Document 4: WO2017/010535A

### SUMMARY OF THE INVENTION

FLT3 plays an important role in the proliferation and differentiation of hematopoietic cells. In normal bone marrow, expression of FLT3 is observed in hematopoietic stem cells, progenitor cells, and the like; however, FLT3 is overexpressed or FLT3 is mutated in hematological cancer, which results in activation of the FLT3 signaling pathway and contributes to the proliferation and malignant transformation of cancer. A new curing method for such diseases is desired.

So far, there have been no reports on the results of the administration of Compound A to patients with acute myeloid leukemia. As a result, it is not known what kind of in-blood kinetics is exhibited by Compound A. In order for Compound A to exhibit drug efficacy thereof to a patient with acute myeloid leukemia, it is important to maintain the concentration equal to or higher than the predetermined in-blood concentration for at least 24 hours. However, even those skilled in the art can not predict at what doses the maintenance of the above concentration can be achieved unless Compound A is administered to a patient with acute myeloid leukemia. In addition, no studies have been carried out so far on the value of the predetermined in-blood concentration of Compound A, which should be maintained for 24 hours or more and required for exhibiting a practical curing effect on a patient with acute myeloid leukemia. An object of the present invention is to provide an antitumor agent for acute myeloid leukemia, which exhibits a practical curing effect on a patient with acute myeloid leukemia.

As a result of diligent studies to solve the above problems, the inventors of the present invention have found that the in-plasma drug concentration which exceeds 40 ng/mL for 24 hours or more can be achieved in a case where the number of administrations per day and the dose per administration are set within the predetermined range. Further, the inventors of the present invention have found that Compound A has an excellent curing effect on acute myeloid leukemia in a case where Compound A is administered under the above conditions. The present invention has been completed based on the above findings.

That is, the present invention provides the followings.
<1> An antitumor agent for acute myeloid leukemia, comprising a compound (Compound A) represented by General Formula [1] or a salt thereof,
   in which in a twice-daily administration, a dose per administration is 25 to 225 mg, or in a thrice-daily administration, the dose per administration is 20 to 150 mg, 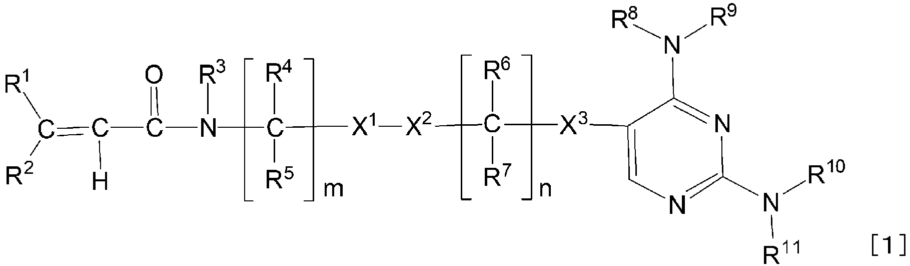 in the formula,
   R¹ represents a hydrogen atom or a C₁₋₆ alkyl group which may be substituted,
   R² represents a hydrogen atom, a C₁₋₆ alkyl group which may be substituted, a C₂₋₆ alkenyl group which may be substituted, or a C₂₋₆ alkynyl group which may be substituted,
   R³ represents a hydrogen atom, a C₁₋₆ alkyl group which may be substituted, a C₂₋₆ alkenyl group which may be substituted, or a C₂₋₆ alkynyl group which may be substituted,
   m represents an integer of 1 to 3,
   m pieces of R⁴'s may be the same or different from each other and represent a hydrogen atom or a C₁₋₆ alkyl group which may be substituted, and one R⁴ selected from the m pieces of R⁴'s may be combined together with R³ to form a C₁₋₆ alkylene group which may be substituted,
   m pieces of R⁵'s may be the same or different from each other and represent a hydrogen atom, a C₁₋₆ alkyl group which may be substituted, a C₂₋₆ alkenyl group which may be substituted, or a C₂₋₆ alkynyl group which may be substituted,
   X¹ represents an oxygen atom, N(R²⁰) (in the formula, R²⁰ represents a hydrogen atom, a C₁₋₆ alkyl group which may be substituted, a C₂₋₆ alkenyl group which may be substituted, or a C₂₋₆ alkynyl group which may be substituted), C(=O), C(=O)-N(R²⁰) (in the formula, R²⁰ has the same meaning as the above), or a bond,
   X² represents a C₁₋₆ alkylene group which may be substituted, a divalent alicyclic hydrocarbon group which may be substituted, or a divalent aromatic hydrocarbon group which may be substituted,
   n represents an integer of 0 to 3,
   n pieces of R⁶'s may be the same or different from each other and represent a hydrogen atom or a C₁₋₆ alkyl group which may be substituted,
   n pieces of R⁷'s may be the same or different from each other and represent a hydrogen atom or a C₁₋₆ alkyl group which may be substituted,
   X³ represents a C₁₋₆ alkylene group which may be substituted, a C₂₋₆ alkenylene group which may be substituted, a C₂₋₆ alkynylene group which may be substituted, or N(R²⁰)-C(=O) (in the formula, R²⁰ has the same meaning as the above),
   R⁸ represents a hydrogen atom, a C₁₋₆ alkyl group which may be substituted, a C₂₋₆ alkenyl group which may be substituted, or a C₂₋₆ alkynyl group which may be substituted,
   R⁹ represents a C₁₋₆ alkyl group which may be substituted, a C₂₋₆ alkenyl group which may be substituted, a C₂₋₆ alkynyl group which may be substituted, or a C₃₋₈ cycloalkyl group which may be substituted,
   R⁸ and R⁹ may be combined together with a nitrogen atom to which R⁸ and R⁹ are bonded, to form a cyclic amino group which may be substituted,
   R¹⁰ represents a hydrogen atom, a C₁₋₆ alkyl group which may be substituted, a C₂₋₆ alkenyl group which may be substituted, or a C₂₋₆ alkynyl group which may be substituted, and
   R¹¹ represents a C₁₋₆ alkyl group which may be substituted, a C₂₋₆ alkenyl group which may be substituted, a C₂₋₆ alkynyl group which may be substituted, a C₃₋₈ cycloalkyl group which may be substituted, an aryl group which may be substituted, or a heterocyclic group which may be substituted.
<2> The antitumor agent according to <1>, in which in the twice-daily administration, the dose per administration is 35 to 150 mg.
<3> The antitumor agent according to <1>, in which in the twice-daily administration, the dose per administration is 35 to 100 mg.
<4> The antitumor agent according to any one of <1> to <3>,
   in which R¹⁰ is a hydrogen atom, and
   X¹ is C(=O)-N(R²⁰) (in the formula, R²⁰ represents a hydrogen atom, a C₁₋₆ alkyl group which may be substituted, a C₂₋₆ alkenyl group which may be substituted, or a C₂₋₆ alkynyl group which may be substituted).
<5> The antitumor agent according to any one of <1> to <4>, in which X³ is a C₂₋₆ alkynylene group which may be substituted.
<6> The antitumor agent according to any one of <1> to <5>, in which the compound represented by General Formula [1] is (S,E)-N-(1-((5-(2-((4-cyanophenyl)amino)-4-(propylamino)pyrimidin-5-yl)-4-pentyn-1-yl)ami no)-1-oxopropan-2-yl)-4-(dimethylamino)-N-methyl-2-butenamide.
<7> The antitumor agent according to <1>, in which in the thrice-daily administration, the dose per administration is 35 to 150 mg.
<8> The antitumor agent according to <1>, in which in the thrice-daily administration, the dose per administration is 35 to 100 mg.
<9> The antitumor agent according to any one of <1> to <8>, in which the antitumor agent is an oral agent.
   (A1) A method for using Compound A for the treatment of acute myeloid leukemia, the method including administrating Compound A to a subject (a mammal including a human) requiring the treatment of acute myeloid leukemia two times a day at a dose per administration of 25 to 225 mg.
   (A2) A method for using Compound A for the treatment of acute myeloid leukemia, the method including administrating Compound A to a subject (a mammal including a human) requiring the treatment of acute myeloid leukemia three times a day at a dose per administration of 20 to 150 mg.
   (B1) A method for treating acute myeloid leukemia, the method including administrating Compound A to a subject (a mammal including a human) requiring the treatment of acute myeloid leukemia two times a day at a dose per administration of 25 to 225 mg.
   (B2) A method for treating acute myeloid leukemia, the method including administrating Compound A to a subject (a mammal including a human) requiring the treatment of acute myeloid leukemia three times a day at a dose per administration of 20 to 150 mg.
   (C1) Use of Compound A for producing an antitumor agent for acute myeloid leukemia, in which in the twice-daily administration, a dose per administration is 25 to 225 mg.
   (C2) Use of Compound A for the producing an antitumor agent for acute myeloid leukemia, in which in the thrice-daily administration, the dose per administration is 20 to 150 mg.
   (D1) Compound A for using in the curing of acute myeloid leukemia, in which in the twice-daily administration, a dose per administration is 25 to 225 mg.
   (D2) Compound A for using in the curing of acute myeloid leukemia, in which in the thrice-daily administration, a dose per administration is 20 to 150 mg.

Compound A has a curing effect on acute myeloid leukemia. That is, according to an aspect of the present invention, an antitumor agent for acute myeloid leukemia that exhibits an effect on acute myeloid leukemia is provided.

### BRIEF DESCRIPTION OF THE DRAWINGS

Fig. 1 is a graph showing a correlation between the in-plasma concentration and the plasma inhibitory activity (PIA) test result for a patient treated with Compound A1.
Fig. 2 is a graph showing simulation results of an administration one time a day (QD).
Fig. 3 is a graph showing simulation results of a twice-daily administration (BID).
Fig. 4 is a graph showing simulation results of a thrice-daily administration (TID).

### DESCRIPTION OF THE PREFERRED EMBODIMENTS

In the present invention, the range represented by "to" includes the values at both ends thereof unless otherwise specified.

The "subject" is a mammal such as a human, a mouse, a monkey, or a domestic animal requiring prevention or curing therefor, and preferably a human requiring prevention or curing therefor.

The "prevention" means the inhibition of onset of a disease, the reduction of the risk of the onset of a disease, or the delay of onset of a disease.

The "curing" means the amelioration or the suppression (the maintenance or delay) of progression of a disease or a state of interest.

The "treatment" means the prevention or the curing of various diseases.

The "tumor" means a benign or malignant tumor.

The "benign tumor" means a tumor in which the morphology of a tumor cell and the sequence of the tumor cell are similar to those of the normal cell from which the tumor cell is derived and which is not invasive or metastatic.

The malignant tumor means a tumor in which the morphology of a tumor cell and the sequence of the tumor cell are different from those of the normal cell from which the tumor cell is derived and which is invasive or metastatic.

The "dose per administration" means a dose of Compound A per administration for a human. The human is preferably an adult.

Examples of the halogen atom include a fluorine atom, a chlorine atom, a bromine atom, and an iodine atom.

Examples of the C₁₋₆ alkyl group include linear or branched C₁₋₆ alkyl groups such as a methyl group, an ethyl group, a propyl group, an isopropyl group, a butyl group, a sec-butyl group, an isobutyl group, a tert-butyl group, a pentyl group, an isopentyl group, and a hexyl group.

Examples of the C₁₋₃ alkyl group include a methyl group, an ethyl group, a propyl group, and an isopropyl group.

Examples of the C₂₋₆ alkenyl group include linear or branched C₂₋₆ alkenyl groups such as a vinyl group, an allyl group, a propenyl group, an isopropenyl group, a butenyl group, an isobutenyl group, a 1,3-butadienyl group, a pentenyl group, and a hexenyl group.

Examples of the C₂₋₆ alkynyl group include linear or branched C₂₋₆ alkynyl groups such as an ethynyl group, a propynyl group, a butynyl group, a pentynyl group, and a hexynyl group.

Examples of the C₃₋₈ cycloalkyl group include C₃₋₈ cycloalkyl groups such as a cyclopropyl group, a cyclobutyl group, a cyclopentyl group, and a cyclohexyl group.

Examples of the aryl group include a phenyl group and a naphthyl group.

Examples of the aryl C₁₋₆ alkyl group include aryl C₁₋₆ alkyl groups such as a benzyl group, a diphenylmethyl group, a trityl group, a phenethyl group, and a naphthylmethyl group.

Examples of the C₁₋₆ alkoxy group include linear, cyclic, or branched C₁₋₆ alkyloxy groups such as a methoxy group, an ethoxy group, a propoxy group, an isopropoxy group, a cyclopropoxy group, a butoxy group, an isobutoxy group, a sec-butoxy group, a tert-butoxy group, a cyclobutoxy group, a pentyloxy group, and hexyloxy group.

Examples of the C₁₋₃ alkoxy group include a methoxy group, an ethoxy group, a propoxy group, and an isopropoxy group.

Examples of the C₁₋₆ alkoxy C₁₋₆ alkyl group include C₁₋₆ alkyloxy C₁₋₆ alkyl groups such as a methoxymethyl group and an 1-ethoxyethyl group.

Examples of the aryl C₁₋₆ alkoxy C₁₋₆ alkyl group include aryl C₁₋₆ alkyloxy C₁₋₆ alkyl groups such as a benzyloxymethyl group and a phenethyloxymethyl group.

Examples of the C₂₋₆ alkanoyl group include linear or branched C₂₋₆ alkanoyl groups such as an acetyl group, a propionyl group, a valeryl group, an isovaleryl group, and a pivaloyl group.

Examples of the aroyl group include a benzoyl group and a naphthoyl group.

Examples of the heterocyclic carbonyl group include a nicotinoyl group, a thenoyl group, a pyrrolidinocarbonyl group, and a furoyl group.

Examples of the (a-substituted) aminoacetyl group include an (a-substituted) aminoacetyl group, the N-terminal of which may be protected and which is derived from an amino acid (examples of the amino acid include glycine, alanine, valine, leucine, isoleucine, serine, threonine, cysteine, methionine, aspartic acid, glutamic acid, asparagine, glutamine, arginine, lysine, histidine, hydroxylysine, phenylalanine, tyrosine, tryptophan, proline, and hydroxyproline).

Examples of the acyl group include a formyl group, a succinyl group, a glutaryl group, a maleoyl group, a phthaloyl group, a C₂₋₆ alkanoyl group, an aroyl group, a heterocyclic carbonyl group, and an (a-substituted) aminoacetyl group.

Examples of the acyl C₁₋₆ alkyl group include acyl C₁₋₆ alkyl groups such as an acetylmethyl group, a benzoylmethyl group, and a 1-benzoylethyl group.

Examples of the acyloxy C₁₋₆ alkyl group include acyloxy C₁₋₆ alkyl groups such as an acetoxymethyl group, a propionyloxymethyl group, a pivaloyloxymethyl group, a benzoyloxymethyl group, and a 1-(benzoyloxy)ethyl group.

Examples of the C₁₋₆ alkoxycarbonyl group include linear or branched C₁₋₆ alkyloxycarbonyl groups such as a methoxycarbonyl group, an ethoxycarbonyl group, an isopropoxycarbonyl group, a tert-butoxycarbonyl group, and a 1,1-dimethylpropoxycarbonyl group.

Examples of the aryl C₁₋₆ alkoxycarbonyl group include aryl C₁₋₆ alkyloxycarbonyl groups such as a benzyloxycarbonyl group and phenethyloxycarbonyl group.

Examples of the aryloxycarbonyl group include a phenyloxycarbonyl group and a naphthyloxycarbonyl group.

Examples of the C₁₋₆ alkylamino group include linear or branched C₁₋₆ alkylamino groups such as a methylamino group, an ethylamino group, a propylamino group, an isopropylamino group, a butylamino group, a sec-butylamino group, a tert-butylamino group, a pentylamino group, and a hexylamino group.

Examples of the di(C₁₋₆ alkyl) amino group include linear or branched di(C₁₋₆ alkyl) amino groups such as a dimethylamino group, a diethylamino group, a dipropylamino group, a diisopropylamino group, a dibutylamino group, a di (tert-butyl) amino group, a dipentylamino group, a dihexylamino group, an (ethyl)(methyl) amino group, and a (methyl)(propyl) amino group.

Examples of the di(C₁₋₃ alkyl) amino group include linear or branched di(C₁₋₃ alkyl) amino groups such as a dimethylamino group, a diethylamino group, a dipropylamino group, a diisopropylamino group, an (ethyl)(methyl) amino group, and a (methyl)(propyl) amino group.

Examples of the C₁₋₆ alkylsulfonyl group include C₁₋₆ alkylsulfonyl groups such as a methylsulfonyl group, an ethylsulfonyl group, and a propylsulfonyl group.

Examples of the arylsulfonyl group include a benzenesulfonyl group, a p-toluenesulfonyl group, and a naphthalenesulfonyl group.

Examples of the C₁₋₆ alkylsulfonyloxy group include C₁₋₆ alkylsulfonyloxy groups such as a methylsulfonyloxy group and an ethylsulfonyloxy group.

Examples of the arylsulfonyloxy group include a benzenesulfonyloxy group and a p-toluenesulfonyloxy group.

Examples of the cyclic amino group include a cyclic amino group which contains one or more nitrogen atoms and may further contain one or more oxygen atoms or sulfur atoms as a heteroatom constituting a ring of a group such as an azetidinyl group, a pyrrolidinyl group, a pyrrolinyl group, a pyrrolyl group, a piperidinyl group, a tetrahydropyridyl group, a homopiperidinyl group, an imidazolidinyl group, an imidazolinyl group, an imidazolyl group, a pyrazolydinyl group, a pyrazolinyl group, a pyrazolyl group, a piperazinyl group, a homopiperazinyl group, a triazolyl group, a tetrazolyl group, a morpholinyl group, a thiomorpholinyl group, a tetrahydroquinolinyl group, a tetrahydroisoquinolinyl group, or a quinuclidinyl group.

Examples of the monocyclic nitrogen-containing heterocyclic group include a monocyclic nitrogen-containing heterocyclic group which contains only a nitrogen atom as a heteroatom constituting a ring of a group such as an azetidinyl group, a pyrrolidinyl group, a pyrrolinyl group, a pyrrolyl group, a piperidyl group, a tetrahydropyridyl group, a pyridyl group, a homopiperidinyl group, an octahydroazosinyl group, an imidazolidinyl group, an imidazolinyl group, an imidazolyl group, a pyrazolydinyl group, a pyrazolinyl group, a pyrazolyl group, a piperazinyl group, a pyrazinyl group, a pyridazinylgroup, a pyrimidinyl group, a homopiperazinyl group, a triazolyl group, or a tetrazolyl group.

Examples of the monocyclic oxygen-containing heterocyclic group include a tetrahydrofuranyl group, a furanyl group, a tetrahydropyranyl group, and a pyranyl group.

Examples of the monocyclic sulfur-containing heterocyclic group include a thienyl group.

Examples of the monocyclic nitrogen-containing and the oxygen-containing heterocyclic group include a monocyclic nitrogen-containing and oxygen-containing heterocyclic group containing only a nitrogen atom and an oxygen atom as a heteroatom constituting a ring of a group such as an oxazolyl group, an isoxazolyl group, an oxadiazolyl group, or a morpholinyl group.

Examples of the monocyclic nitrogen-containing and sulfur-containing heterocyclic group include a monocyclic nitrogen-containing and sulfur-containing heterocyclic group containing only a nitrogen atom and a sulfur atom as a heteroatom constituting a ring of a group such as a thiazolyl group, an isothiazolyl group, a thiadiazolyl group, a thiomorpholinyl group, a 1-oxidothiomorpholinyl group, or a 1,1-dioxidothiomorpholinyl group.

Examples of the monocyclic heterocyclic group include a monocyclic nitrogen-containing heterocyclic group, a monocyclic oxygen-containing heterocyclic group, a monocyclic sulfur-containing heterocyclic group, a monocyclic nitrogen-containing and oxygen-containing heterocyclic group, and a monocyclic nitrogen-containing and sulfur-containing heterocyclic group.

Examples of the bicyclic nitrogen-containing heterocyclic group include a bicyclic nitrogen-containing heterocyclic group containing only a nitrogen atom as a heteroatom constituting a ring of a group such as an indolinyl group, an indolyl group, an isoindolinyl group, an isoindolyl group, a benzimidazolyl group, indazolyl group, a benzotriazolyl group, a pyrazolopyridinyl group, a quinolyl group, a tetrahydroquinolinyl group, a quinolyl group, a tetrahydroisoquinolinyl group, an isoquinolinyl group, a quinolizinyl group, a cinnolinyl group, a phthalazinyl group, a quinazolinyl group, a dihydroquinoxalinyl group, a quinoxalinyl group, a naphthyridinyl group, a purinyl group, a pteridinyl group, or a quinuclidinyl group.

Examples of the bicyclic oxygen-containing heterocyclic group include a bicyclic oxygen-containing heterocyclic group containing only an oxygen atom as a heteroatom constituting a ring of a group such as a 2,3-dihydrobenzofuranyl group, a benzofuranyl group, an isobenzofuranyl group, a chromanyl group, a chromenyl group, an isochromanyl group, a 1,3-benzodioxolyl group, a 1,3-benzodioxanyl group, or a 1,4-benzodioxanyl group.

Examples of the bicyclic sulfur-containing heterocyclic group include a bicyclic sulfur-containing heterocyclic group containing only a sulfur atom as a heteroatom constituting a ring of a group such as a 2,3-dihydrobenzothienyl group or a benzothienyl group.

Examples of the bicyclic nitrogen-containing and oxygen-containing heterocyclic group include a bicyclic nitrogen-containing and oxygen-containing heterocyclic group containing only a nitrogen atom and an oxygen atom as heteroatoms constituting a ring of a group such as a benzoxazolyl group, a benzisoxazolyl group, a benzoxadiazolyl group, a benzomorpholinyl group, a dihydropyranopyridyl group, a dihydrodioxynopyridyl group, or a dihydropyridooxadinyl group.

Examples of the bicyclic nitrogen-containing and sulfur-containing heterocyclic group include a bicyclic nitrogen-containing and sulfur-containing heterocyclic group containing a nitrogen atom and a sulfur atom as heteroatoms constituting a ring of a group such as a benzothiazolyl group, a benzisothiazolyl group, or a benzothiadiazolyl group.

Examples of the bicyclic heterocyclic group include a bicyclic nitrogen-containing heterocyclic group, a bicyclic oxygen-containing heterocyclic group, a bicyclic sulfur-containing heterocyclic group, a bicyclic nitrogen-containing and oxygen-containing heterocyclic group, and a bicyclic nitrogen-containing and sulfur-containing heterocyclic group.

Examples of the heterocyclic group include a monocyclic heterocyclic group and a bicyclic heterocyclic group.

Examples of the C₁₋₆ alkylene group include linear or branched C₁₋₆ alkylene groups such as a methylene group, an ethylene group, a propylene group, a butylene group, and a hexylene group.

Examples of the C₁₋₃ alkylene group include a methylene group, an ethylene group, and a propylene group.

Examples of the C₂₋₆ alkenylene group include linear or branched C₂₋₆ alkenylene groups such as a vinylene group, a propenylene group, a butenylene, and a pentenylene group.

Examples of the C₂₋₆ alkynylene group include linear or branched C₂₋₆ alkynylene groups such as an ethynylene group, a propynylene group, a butynylene group, and a pentynylene group.

Examples of the divalent alicyclic hydrocarbon group include a group formed by removing two hydrogen atoms from an alicyclic hydrocarbon ring, such as a 1,2-cyclobutylene group, a 1,3-cyclobutylene group, a 1,2-cyclopentylene group, a 1,3-cyclopentylene group, a 1,2-cyclohexylene group, a 1,3-cyclohexylene group, a 1,4-cyclohexylene group, a bicyclo(3.2.1) octylene group, a bicyclo(2.2.0) hexylene group, or a bicyclo(5.2.0) nonylene group.

Examples of the divalent aromatic hydrocarbon group include a group formed by removing two hydrogen atoms from an aromatic hydrocarbon ring, such as a phenylene group, an indenylene group, a naphthylene group, a fluorenylene group, a phenanthrenylene group, anthrylene group, or a pyrenylene group.

Examples of the silyl group include a trimethylsilyl group, a triethylsilyl group, and tributylsilyl group.

The amino protecting group includes all groups that can be used as the typical protecting group for an amino group, and examples thereof include groups described in T. W. Greene et al., Protective Groups in Organic Synthesis, 4th Edition, pp. 696 to 926, 2007, John Wiley & Sons Inc. Specific examples thereof include an aryl C₁₋₆ alkyl group, a C₁₋₆ alkoxy C₁₋₆ alkyl group, an acyl group, a C₁₋₆ alkoxycarbonyl group, an aryl C₁₋₆ alkoxycarbonyl group, an aryloxycarbonyl group, a C₁₋₆ alkylsulfonyl group, an arylsulfonyl group, and a silyl group.

The imino protecting group includes all groups that can be used as the typical protecting group for an imino group, and examples thereof include groups described in T. W. Greene et al., Protective Groups in Organic Synthesis, 4th Edition, pp. 696 to 868, 2007, John Wiley & Sons Inc. Specific examples thereof include an aryl C₁₋₆ alkyl group, a C₁₋₆ alkoxy C₁₋₆ alkyl group, an acyl group, a C₁₋₆ alkoxycarbonyl group, an aryl C₁₋₆ alkoxycarbonyl group, an aryloxycarbonyl group, a C₁₋₆ alkylsulfonyl group, an arylsulfonyl group, and a silyl group.

The hydroxyl protecting group includes all groups that can be used as the typical protecting group for a hydroxyl group, and examples thereof include groups described in T. W. Greene et al., Protective Groups in Organic Synthesis, 4th Edition, pp. 16 to 299, 2007, John Wiley & Sons Inc. Specific examples thereof include a C₁₋₆ alkyl group, a C₂₋₆ alkenyl group, an aryl C₁₋₆ alkyl group, a C₁₋₆ alkoxy C₁₋₆ alkyl group, an aryl C₁₋₆ alkoxy C₁₋₆ alkyl group, an acyl groups, a C₁₋₆ alkoxycarbonyl group, an aryl C₁₋₆ alkoxycarbonyl group, a C₁₋₆ alkylsulfonyl group, an arylsulfonyl group, a silyl group, a tetrahydrofuranyl group, and a tetrahydropyranyl groups.

The carboxyl protecting group includes all groups that can be used as the typical protecting group for a carboxyl group, and examples thereof include groups described in T. W. Greene et al., Protective Groups in Organic Synthesis, 4th Edition, pp. 533 to 643, 2007, John Wiley & Sons Inc. Specific examples thereof include a C₁₋₆ alkyl group, a C₂₋₆ alkenyl group, an aryl group, an aryl C₁₋₆ alkyl group, a C₁₋₆ alkoxy C₁₋₆ alkyl group, an aryl C₁₋₆ alkoxy C₁₋₆ alkyl group, an acyl C₁₋₆ alkyl group, an acyloxy C₁₋₆ alkyl group, and a silyl group.

### [Compound of General Formula [1] and salt thereof]

Compound A in the present invention is a compound represented by General Formula [1] and a salt thereof.

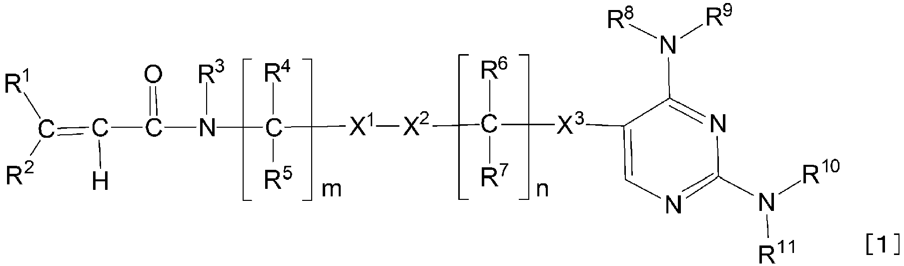

(In the formula, R¹, R², R³, R⁴, R⁵, R⁶, R⁷, R⁸, R⁹, R¹⁰, R¹¹, X¹, X², X³, m, and n have the same meanings as those described above.)

R¹ is a hydrogen atom or a C₁₋₆ alkyl group which may be substituted and preferably a hydrogen atom.

In any case where other substituents are any substituents, the C₁₋₆ alkyl group as R¹ may be substituted with one or more groups selected from a halogen atom, a cyano group, an amino group which may be protected, and a hydroxyl group which may be protected.

The C₁₋₆ alkyl group of the C₁₋₆ alkyl group which may be substituted, as R¹, is preferably a C₁₋₃ alkyl group.

R² is a hydrogen atom, a C₁₋₆ alkyl group which may be substituted, a C₂₋₆ alkenyl group which may be substituted, or a C₂₋₆ alkynyl group which may be substituted, preferably a hydrogen atom or a C₁₋₆ alkyl group which may be substituted, and more preferably a C₁₋₆ alkyl group which may be substituted.

In any case where other substituents are any substituents, the C₁₋₆ alkyl group, the C₂₋₆ alkenyl group, or the C₂₋₆ alkynyl group, as R², may be substituted with one or more groups selected from a C₁₋₆ alkylamino group which may be substituted with one or more groups selected from the substituent group A, a di(C₁₋₆ alkyl) amino group which may be substituted with one or more groups selected from the substituent group A, and a heterocyclic group which may be substituted with one or more groups selected from the substituent group A.

The substituent group A: a halogen atom, a cyano group, an amino group which may be protected, a hydroxyl group which may be protected, a C₁₋₆ alkyl group which may be substituted with one or more groups selected from the substituent group B, a C₃₋₈ cycloalkyl group which may be substituted with one or more groups selected from the substituent group B, an aryl group which may be substituted with one or more groups selected from the substituent group B, a C₁₋₆ alkoxy group which may be substituted with one or more groups selected from the substituent group B, a C₁₋₆ alkylamino group which may be substituted with one or more groups selected from the substituent group B, a di(C₁₋₆ alkyl) amino group which may be substituted with one or more groups selected from the substituent group B, or a heterocyclic group which may be substituted with one or more groups selected from the substituent group B, and an oxo group.

The substituent group B: a halogen atom, a cyano group, an amino group which may be protected, a hydroxyl group which may be protected, a C₁₋₆ alkyl group which may be substituted with a halogen atom or a hydroxyl group, a C₁₋₆ alkoxy group which may be substituted with a halogen atom or a hydroxyl group, an aryl group, a heterocyclic group, and an oxo group.

The C₁₋₆ alkyl group which may be substituted, as R², is preferably a C₁₋₆ alkyl group which is substituted with a di(C₁₋₆ alkyl) amino group, more preferably a C₁₋₃ alkyl group which is substituted with a di(C₁₋₃ alkyl) amino group, and still more preferably a dimethylaminomethyl group.

The C₁₋₆ alkyl group of the C₁₋₆ alkyl group which may be substituted, as R², is preferably a C₁₋₃ alkyl group and more preferably a methyl group.

The substituent of each of the C₁₋₆ alkyl group which may be substituted, the C₂₋₆ alkenyl group which may be substituted, or the C₂₋₆ alkynyl group which may be substituted, as R², is preferably a di(C₁₋₆ alkyl) amino group which may be substituted with one or more groups selected from the substituent group A-1 or a heterocyclic group which may be substituted with one or more groups selected from the substituent group A-1, and more preferably a di(C₁₋₆ alkyl) amino group which may be substituted with one or more groups selected from the substituent group A-1.

The di(C₁₋₆ alkyl) amino group of the di(C₁₋₆ alkyl) amino group which may be substituted with one or more groups selected from the substituent group A-1 is preferably a di(C₁₋₃ alkyl) amino group and more preferably a dimethylamino group.

The heterocyclic group of the heterocyclic group which may be substituted with one or more groups selected from the substituent group A-1 is preferably an azetidinyl group, a piperazinyl group, or a morpholinyl group.

The substituent group A-1: a halogen atom, a hydroxyl group which may be protected, and a C₁₋₆ alkyl group which may be substituted with a hydroxyl group.

R³ is a hydrogen atom, a C₁₋₆ alkyl group which may be substituted, a C₂₋₆ alkenyl group which may be substituted, or a C₂₋₆ alkynyl group which may be substituted, preferably a hydrogen atom or a C₁₋₆ alkyl group, and more preferably a C₁₋₆ alkyl group.

In any case where other substituents are any substituents, the C₁₋₆ alkyl group, the C₂₋₆ alkenyl group, or the C₂₋₆ alkynyl group, as R³, may be substituted with one or more groups selected from a halogen atom, a cyano group, an amino group which may be protected, a hydroxyl group which may be protected, an aryl group which may be substituted with one or more groups selected from the substituent group A, and a heterocyclic group which may be substituted with one or more groups selected from the substituent group A.

The C₁₋₆ alkyl group of the C₁₋₆ alkyl group which may be substituted, as R³, is preferably a C₁₋₃ alkyl group and more preferably a methyl group.

m is an integer of 1 to 3, preferably an integer of 1 or 2, and more preferably an integer of 1.

m pieces of R⁴'s are the same or different from each other, are a hydrogen atom or a C₁₋₆ alkyl group which may be substituted, and are preferably a hydrogen atom.

In any case where other substituents are any substituents, the C₁₋₆ alkyl group as R⁴ may be substituted with one or more groups selected from a halogen atom, a cyano group, an amino group which may be protected, and a hydroxyl group which may be protected.

One R⁴ selected from the m pieces of R⁴'s may be combined together with R³ to form a C₁₋₆ alkylene group which may be substituted, and the C₁₋₆ alkylene group of the C₁₋₆ alkylene group which may be substituted is preferably a C₁₋₃ alkylene group and more preferably a propylene group. The substituent of the C₁₋₆ alkylene group which may be substituted is preferably a halogen atom, a hydroxyl group, or a C₁₋₃ alkoxy group, more preferably a fluorine atom, a hydroxyl group, or a methoxy group, and still more preferably a fluorine atom or a methoxy group.

m pieces of R⁵'s are the same or different from each other, are a hydrogen atom, a C₁₋₆ alkyl group which may be substituted, a C₂₋₆ alkenyl group which may be substituted, or a C₂₋₆ alkynyl group which may be substituted, and preferably a C₁₋₆ alkyl group which may be substituted.

In any case where other substituents are any substituents, the C₁₋₆ alkyl group, the C₂₋₆ alkenyl group which may be substituted, or the C₂₋₆ alkynyl group which may be substituted, as R⁵, may be substituted with one or more groups selected from a halogen atom, a cyano group, an amino group which may be protected, and a hydroxyl group which may be protected.

The C₁₋₆ alkyl group of the C₁₋₆ alkyl group which may be substituted, as R⁵, is preferably a C₁₋₃ alkyl group and more preferably a methyl group.

n is an integer of 0 to 3, preferably an integer of 0 or 1, and more preferably an integer of 0.

n pieces of R⁶'s are the same or different from each other and are a hydrogen atom or a C₁₋₆ alkyl group which may be substituted, preferably a hydrogen atom or a C₁₋₆ alkyl group, and ore preferably a hydrogen atom.

n pieces of R⁷'s are the same or different from each other and are a hydrogen atom or a C₁₋₆ alkyl group, preferably a hydrogen atom or a C₁₋₆ alkyl group, and more preferably a hydrogen atom.

In any case where other substituents are any substituents, the C₁₋₆ alkyl group as R⁶ and R⁷ may be substituted with a halogen atom, a cyano group, an amino group which may be protected, or a hydroxyl group which may be protected.

R⁸ is a hydrogen atom, a C₁₋₆ alkyl group which may be substituted, a C₂₋₆ alkenyl group which may be substituted, or a C₂₋₆ alkynyl group which may be substituted, and preferably a hydrogen atom.

In any case where other substituents are any substituents, the C₁₋₆ alkyl group, the C₂₋₆ alkenyl group, or the C₂₋₆ alkynyl group, as R⁶, may be substituted with one or more groups selected from a halogen atom, a cyano group, an amino group which may be protected, and a hydroxyl group which may be protected.

R⁹ is a C₁₋₆ alkyl group which may be substituted, a C₂₋₆ alkenyl group which may be substituted, a C₂₋₆ alkynyl group which may be substituted, or a C₃₋₈ cycloalkyl group which may be substituted, preferably a C₁₋₆ alkyl group which may be substituted or a C₃₋₈ cycloalkyl group which may be substituted, and more preferably a C₁₋₆ alkyl group which may be substituted.

In any case where other substituents are any substituents, the C₁₋₆ alkyl group, the C₂₋₆ alkenyl group, the C₂₋₆ alkynyl group, or the C₃₋₈ cycloalkyl group, as R⁹, may be substituted with one or more groups selected from a halogen atom, a cyano group, an amino group which may be protected, a hydroxyl group which may be protected, and a C₁₋₆ alkoxy group which may be substituted with one or more groups selected from the substituent group A.

The C₁₋₆ alkyl group which may be substituted, as R⁹, is preferably a C₁₋₆ alkyl group which may be substituted.

The C₁₋₆ alkyl group of the C₁₋₆ alkyl group which may be substituted, as R⁹, is preferably a C₁₋₃ alkyl group.

The substituent of the C₁₋₆ alkyl group as R⁹, which may be substituted, is preferably a halogen atom or a C₁₋₃ alkoxy group and more preferably a methoxy group.

R⁸ and R⁹ may be combined together with a nitrogen atom to which R⁸ and R⁹ are bonded, to form a cyclic amino group which may be substituted, where the cyclic amino group which may be substituted is preferably a morpholinyl group.

In any case where other substituents are any substituents, the cyclic amino group formed by combining R⁸ and R⁹ together with the nitrogen atom to which R⁸ and R⁹ are bonded may be substituted with one or more groups selected from a halogen atom, a cyano group, an amino group which may be protected, a hydroxyl group which may be protected, and an oxo group.

R¹⁰ is a hydrogen atom, a C₁₋₆ alkyl group which may be substituted, a C₂₋₆ alkenyl group which may be substituted, or a C₂₋₆ alkynyl group which may be substituted, and preferably a hydrogen atom.

In any case where other substituents are any substituents, the C₁₋₆ alkyl group, the C₂₋₆ alkenyl group, or the C₂₋₆ alkynyl group, as R¹⁰, may be substituted with one or more groups selected from a halogen atom, a cyano group, an amino group which may be protected, a hydroxyl group which may be protected, and a C₁₋₆ alkoxy group which may be substituted with one or more groups selected from the substituent group A.

R¹¹ is a C₁₋₆ alkyl group which may be substituted, a C₂₋₆ alkenyl group which may be substituted, a C₂₋₆ alkynyl group which may be substituted, a C₃₋₈ cycloalkyl group which may be substituted, an aryl group which may be substituted, or a heterocyclic group which may be substituted, preferably a C₁₋₆ alkyl group which may be substituted, an aryl group which may be substituted, or a heterocyclic group which may be substituted, more preferably an aryl group which may be substituted or a heterocyclic group which may be substituted, and still more preferably an aryl group which may be substituted.

In any case where other substituents are any substituents, the C₁₋₆ alkyl group, the C₂₋₆ alkenyl group, the C₂₋₆ alkynyl group, the C₃₋₈ cycloalkyl group, the aryl group, or the heterocyclic group, as R¹¹, may be substituted with one or more groups selected from a halogen atom, a cyano group, an amino group which may be protected, a hydroxyl group which may be protected, and a C₁₋₆ alkoxy group which may be substituted with one or more groups selected from the substituent group A.

The substituent of each, as R¹¹, of the C₁₋₆ alkyl group which may be substituted, the C₃₋₈ cycloalkyl group which may be substituted, the aryl group which may be substituted, or the heterocyclic group which may be substituted, is preferably a C₁₋₆ alkoxy group which may be substituted with one or more groups selected from the substituent group A-2.

The substituent group A-2: a halogen atom, a C₁₋₆ alkyl group, a C₃₋₈ cycloalkyl group, a C₁₋₆ alkoxy group, and a heterocyclic group.

The C₁₋₆ alkyl group which may be substituted, as R¹¹, is preferably a C₁₋₆ alkyl group which is substituted, more preferably a C₁₋₃ alkyl group which is substituted, and still more preferably an ethyl group which is substituted.

In a case where R¹¹ is a C₁₋₆ alkyl group which is substituted, the substituent of the C₁₋₆ alkyl group is preferably a heterocyclic group, more preferably a pyridyl group, a pyrrolidinyl group, or a morpholinyl group.

The aryl group which may be substituted, as R¹¹, is preferably an aryl group which is substituted, more preferably a phenyl group which is substituted.

In a case where R¹¹ is a phenyl group which is substituted, the substituent of the phenyl group is preferably a halogen atom, a cyano group, or a carbamoyl group and more preferably a fluorine atom or a cyano group.

In a case where R¹¹ is a phenyl group which is substituted, the phenyl group preferably has no substituent at the o-position but has a substituent at the m-position or the p-position, and more preferably has a substituent only at the p-position.

The preferred substituent at the m-position or p-position is as described above.

The heterocyclic group which may be substituted, as R¹¹, is preferably a pyridyl group which may be substituted, an indazolyl group which may be substituted, a pyrazolopyridinyl group which may be substituted, or an isoquinolyl group which may be substituted.

X¹ is an oxygen atom, N(R²⁰) (in the formula, R²⁰ has the same meaning as the above), C(=O), C(=O)-N(R²⁰) (in the formula, R²⁰ has the same meaning as the above), or a bond and is preferably C(=O)-N(R²⁰) (in the formula, R²⁰ has the same meaning as the above).

R²⁰ is a hydrogen atom, a C₁₋₆ alkyl group which may be substituted, a C₂₋₆ alkenyl group which may be substituted, or a C₂₋₆ alkynyl group which may be substituted and is preferably a hydrogen atom.

In any case where other substituents are any substituents, the C₁₋₆ alkyl group, the C₂₋₆ alkenyl group, or the C₂₋₆ alkynyl group, as R²⁰, may be substituted with one or more groups selected from a halogen atom, a cyano group, an amino group which may be protected, and a hydroxyl group which may be protected.

X² is a C₁₋₆ alkylene group which may be substituted, a divalent alicyclic hydrocarbon group which may be substituted, or a divalent aromatic hydrocarbon group which may be substituted and preferably a C₁₋₆ alkylene group which may be substituted or a divalent alicyclic hydrocarbon group which may be substituted.

In any case where other substituents are any substituents, the C₁₋₆ alkylene group, the divalent alicyclic hydrocarbon group, or the divalent aromatic hydrocarbon group, as X², may be substituted with one or more groups selected from a halogen atom, a cyano group, an amino group which may be protected, and a hydroxyl group which may be protected.

The C₁₋₆ alkylene group which may be substituted, as X², is preferably a C₁₋₆ alkylene group which is unsubstituted.

The C₁₋₆ alkylene group of the C₁₋₆ alkylene group which may be substituted, as X², is preferably a methylene group, an ethylene group, or a trimethylene and more preferably a trimethylene group.

The substituent of the C₁₋₆ alkylene group which may be substituted, as X², is preferably a C₁₋₆ alkyl group, more preferably a C₁₋₃ alkyl group, and still more preferably an ethyl group.

The divalent alicyclic hydrocarbon group which may be substituted, as X², is preferably a divalent alicyclic hydrocarbon group which is unsubstituted.

The divalent alicyclic hydrocarbon group of the divalent alicyclic hydrocarbon group which may be substituted, as X², is preferably cyclobutylene group or cyclohexylene group and more preferably a cyclobutylene group.

In a case of being a cyclobutylene group, X² is preferably a cyclobutylene group represented by Formula [2] 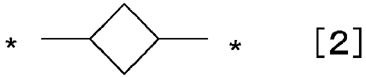 (in the formula, * indicates a bonding position) and more preferably a cyclobutylene group represented by Formula [3] 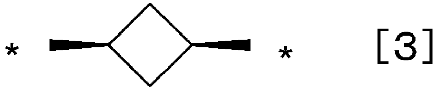 (in the formula, * indicates a bonding position).

In a case of being a cyclohexylene group, X² is preferably a cyclohexylene group represented by Formula [4] 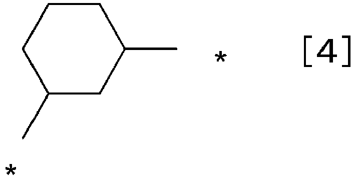 (in the formula, * indicates a bonding position).

The divalent aromatic hydrocarbon group of the divalent aromatic hydrocarbon group which may be substituted, as X², is preferably a phenylene group.

In a case of being a phenylene group, X² is preferably a phenylene group represented by Formula [5] 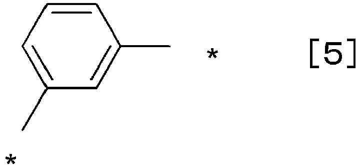 (In the formula, * indicates a bonding position.)

The substituent of the divalent aromatic hydrocarbon group as X², which may be substituted, is preferably a halogen atom or a C₁₋₆ alkyl group.

In a case of being a halogen atom, the substituent is preferably a chlorine atom.

In a case of being a C₁₋₆ alkyl group, the substituent is preferably a C₁₋₃ alkyl group and more preferably a methyl group.

X³ is a C₁₋₆ alkylene group which may be substituted, a C₂₋₆ alkenylene group which may be substituted, a C₂₋₆ alkynylene group which may be substituted, or N(R²⁰)-C(=O) (in the formula, R²⁰ has the same meaning as the above), preferably a C₂₋₆ alkynylene group which may be substituted or N(R²⁰)-C(=O)) (in the formula, R²⁰ has the same meaning as the above), and more preferably a C₂₋₆ alkynylene group which may be substituted.

In any case where other substituents are any substituents, the C₁₋₆ alkylene group, the C₂₋₆ alkenylene group, or the C₂₋₆ alkynylene group, as X³, may be substituted with one or more groups selected from a halogen atom, a cyano group, an amino group which may be protected, and a hydroxyl group which may be protected.

The C₂₋₆ alkynylene group of the C₂₋₆ alkynylene group which may be substituted, as X³, is preferably an ethynylene group.

Examples of the salt of the compound of General Formula [1] include a salt of a generally known basic group such as an amino group and a salt of a generally known acidic group such as a hydroxyl group or a carboxyl group.

Examples of the salt of the basic group include a salt with a mineral acid such as hydrochloric acid, hydrobromic acid, nitric acid, or sulfuric acid; a salt with an organic carboxylic acid such as formic acid, acetic acid, citric acid, oxalic acid, fumaric acid, maleic acid, succinic acid, malic acid, tartaric acid, aspartic acid, trichloroacetic acid, or trifluoroacetic acid; and a salt with a sulfonic acid such as methanesulfonic acid, benzenesulfonic acid, p-toluenesulfonic acid, mesitylenesulfonic acid, or naphthalenesulfonic acid.

Among the salts described above, examples of the preferred salt include a pharmacologically acceptable salt. A more preferred salt is a succinate salt.

The salt may be an anhydride, a hydrate, or a solvate.

Specific examples of Compound A (the compound represented by General Formula [1]) include the compounds described in Tables 1-1 to 1-4 after paragraph 0130 of Patent Document 3.

The particularly preferred compound is (S,E)-N-(1-((5-(2-((4-cyanophenyl)amino)-4-(propylamino)pyrimidin-5-yl)-4-pentyn-1-yl)ami no)-1-oxopropan-2-yl)-4-(dimethylamino)-N-methyl-2-butenamide, and this compound is particularly referred to as Compound A1 in the present specification.

Compound A1 may also be referred to as (S,E)-N-{1-[(5-{2-[(4-cyanophenyl)amino]-4-(propylamino)pyrimidin-5-yl}pent-4-yn-1-yl)a mino]-1-oxopropan-2-yl}-4-(dimethylamino)-N-methylbut-2-enamide.

Compound A may be a compound or a salt thereof, which is represented by General Formula [1] of Patent Document 1, and the description thereof can be referred to and taken into consideration, the contents of which are incorporated in the present specification.

In addition, other preferred compounds of Compound A include the followings.
(S,E)-4-(dimethylamino)-N-(1-((5-(2-((3-fluorophenyl)amino)-4-(propylamino)pyrim idin-5-yl)pent-4-yn-1-yl)amino)-1-oxopropan-2-yl)-N-methylbut-2-enamide (Compound 34 of Patent Document 3),
(E)-4-(dimethylamino)-N-((S)-1-(((1s,3R)-3-((2-((3-fluorophenyl)amino)-4-(propyla mino)pyrimidin-5-yl)ethynyl)cyclobutyl)amino)-1-oxopropan-2-yl)-N-methylbut-2-enamide (Compound 39 of Patent Document 3),
(E)-N-((S)-1-(((1s,3R)-3-((4-(cyclopropylamino)-2-((4-fluorophenyl)amino)pyrimidi n-5-yl)ethynyl)cyclobutyl)amino)-1-oxopropan-2-yl)-4-(dimethylamino)-N-methylbut-2-enam ide (Compound 40 of Patent Document 3), and
(E)-4-(dimethylamino)-N-((S)-1-(((1s,3R)-3-((2-((4-fluorophenyl)amino)-4-(methyla mino)pyrimidin-5-yl)ethynyl)cyclobutyl)amino)-1-oxopropan-2-yl)-N-methylbut-2-enamide (Compound 41 of Patent Document 3).

Next, a method for producing Compound A will be described. Compound A can be produced, for example, by the method disclosed in Patent Document 4. Further, a salt of Compound A can be produced by the method disclosed in Patent Document 2.

Next, a pharmaceutical composition containing Compound A will be described. Composition A may be administered to a patient in the form of a pharmaceutical composition. The pharmaceutical composition contains Compound A and at least one or more kinds of the group of celluloses, sugars, and a sugar alcohol.

It is preferable for the pharmaceutical composition to have an excellent dissolution property; for example, in the dissolution test of the 17th revised Japanese Pharmacopoeia dissolution test method (the paddle method), the dissolution rate after 30 minutes is preferably 85% by mass or more, where the dissolution test is carried out using an acetate buffer solution having a pH of 4.5 as the test solution under the condition of the rotation rate of 50 rotations/minute.

Examples of celluloses include a water-insoluble cellulose derivative and a water-soluble cellulose derivative, and preferred examples thereof include a water-insoluble cellulose derivative.

Examples of the water-soluble cellulose derivative include hypromellose, hydroxypropyl cellulose, and sodium carboxymethyl cellulose.

Examples of the water-insoluble cellulose derivative include a crystalline cellulose, a powdered cellulose, ethyl cellulose, carmellose calcium, croscarmellose sodium, and hydroxypropyl cellulose with low-substitution degree, preferred examples thereof include a crystalline cellulose and a powdered cellulose, and more preferred examples thereof include a crystalline cellulose.

Examples of the crystalline cellulose include CEOLUS KG-1000 (Asahi Kasei Corporation), CEOLUS PH101 (Asahi Kasei Corporation), and CEOLUS PH-F20JP (Asahi Kasei Corporation), and preferred examples thereof include CEOLUS KG-1000 (Asahi Kasei Corporation).

Examples of the sugars include white sugar, lactose, maltose, and glucose, preferred examples thereof include lactose and maltose, and more preferred examples thereof include lactose.

Further, examples of the lactose include a lactose hydrate, anhydrous lactose, a spray-dried lactose hydrate, and a granulated lactose hydrate.

Examples of the sugar alcohol include mannitol, erythritol, and xylitol, preferred examples thereof include mannitol and erythritol, and more preferred examples thereof include erythritol.

These celluloses, sugars, and sugar alcohol may be used alone or in a combination of two or more thereof.

The combined content of celluloses, sugars, and sugar alcohol is preferably 5% to 95%, more preferably 10% to 90%, and still more preferably 15% to 75%, with respect to the composition.

The pharmaceutical composition may contain a lubricant. Examples of the lubricant used in the present invention include magnesium stearate, sodium stearyl fumarate, and hardened oil, and more preferred examples thereof include magnesium stearate and sodium stearyl fumarate.

Examples of magnesium stearate include Partec™ LUB MST (Merck KGaA), magnesium stearate (vegetable) (Taihei Chemical Industrial Co., Ltd.), and Japanese Pharmacopeia Magnesium stearate JPM (Sakai Chemical Industry Co., Ltd.).

Examples of sodium stearyl fumarate include PRUV (JRS PHARMA).

The content of the lubricant is preferably 0.05% to 35%, more preferably 0.5% to 20%, and still more preferably 1% to 15%, with respect to the composition.

For the pharmaceutical composition, a pharmaceutically acceptable formulation auxiliary agent can be used.

Examples of the formulation auxiliary agent include a disintegrating agent, a binder, a taste modifier, a coloring agent, a flavoring agent, a surfactant, a coating agent, and a plasticizer.

The pharmaceutical composition can be used as a pharmaceutical formulation such as a tablet, a hard capsule agent, a granular agent, a fine granular agent, a powdery agent, a fast-disintegrating tablet, a pharmaceutical formulation dissoluble at use, a dry syrup, or a powder formulation, by appropriately using an excipient, a lubricant, and a pharmaceutical formulation auxiliary agent, which are pharmaceutically acceptable. A hard capsule agent or a tablet is preferable, and a hard capsule agent is more preferable. Examples of the hard capsule include a hard capsule manufactured using gelatin, hypromellose, pullulan, or the like as a raw material, and preferred examples thereof include a hard capsule manufactured using hypromellose. Examples thereof include Vcaps Plus (Lonza Group AG) and Quali-V (Qualicaps Co., Ltd.). The size of the hard capsule agent is preferably No. 0 to No. 4 and more preferably No. 2 to No. 4.

The production of the pharmaceutical composition is not particularly limited and may be carried out by a conventional method.

Examples of the method for producing the pharmaceutical composition include a method of filling a hard capsule with a mixture of raw materials or tableting a mixture of raw materials. Another example thereof includes a method of granulating a mixture of raw materials and filling a hard capsule with the obtained granulated product or tableting the obtained granulated product.

### (Antitumor agent for acute myeloid leukemia)

Compound A according to the embodiment of the present invention can be used as an antitumor agent or as an active ingredient of a pharmaceutical composition. According to the present invention, an antitumor agent for acute myeloid leukemia containing Compound A is provided. The following administration may be carried out before, during, or after heaving a meal but is preferably before a meal. It is preferable to be administered after 1 hour or more has passed from the meal before administration, more preferably after 2 hours or more, and particularly preferably after 6 hours or more. It is preferable to have a meal after 1 hour or more after administration and more preferably after 2 hours or more.

The present invention relates to an antitumor agent for acute myeloid leukemia, containing Compound A, in which in the twice-daily administration (the BID administration), the dose per administration is 25 to 225 mg. In a case where the dose is set in such a range, the curing effect as an antitumor agent can be maximized while minimizing side effects.

In the BID administration, the dose per administration is preferably 35 to 150 mg, more preferably 35 to 100 mg, and still more preferably 40 to 90 mg.

In the BID administration, the lower limit value of the dose per administration is 25 mg, preferably 35 mg, more preferably 50 mg, and particularly preferably 60 mg.

In the BID administration, the upper limit value of the dose per administration is 225 mg, preferably 200 mg, more preferably 150 mg, and particularly preferably 130 mg.

The present invention relates to an antitumor agent for acute myeloid leukemia, containing Compound A, in which in the thrice-daily administration (the TID administration), the dose per administration is 20 to 150 mg. In a case where the dose is set in such a range, the curing effect as an antitumor agent can be maximized while minimizing side effects.

In the TID administration, the dose per administration is preferably 35 to 150 mg and more preferably 35 to 100 mg.

In the TID administration, the lower limit value of the dose per administration is 20 mg, preferably 35 mg, more preferably 50 mg, and particularly preferably 60 mg.

In the TID administration, the upper limit value of the dose per administration is 200 mg, preferably 100 mg, more preferably 80 mg, and particularly preferably 50 mg.

The present invention relates to an antitumor agent for acute myeloid leukemia, containing Compound A, in which in the administration one time a day (the QD administration) before having a meal, the dose per administration is 50 to 300 mg. In a case where the dose is set in such a range, the curing effect as an antitumor agent can be maximized while minimizing side effects. In QD administration, in order to maximize the curing effect as an antitumor agent, it is better to be administered every 24 hours as a guide when a patient is in a fasting state. Accordingly, it is preferable to be administered after 1 hour or more has passed from the meal before administration, more preferably after 2 hours or more, and particularly preferably after 6 hours or more. It is preferable to have a meal after 1 hour or more after administration and more preferably after 2 hours or more. In the case of the QD administration, it is preferable to be administered before breakfast.

In the QD administration, the dose per administration is preferably 75 to 275 mg, more preferably 75 to 250 mg, still more preferably 75 to 225 mg, even still more preferably 100 to 225 mg, and even further still more preferably 100 to 150 mg.

In the QD administration, the lower limit value of the dose per administration is 50 mg, preferably 75 mg, and more preferably 100 mg.

In the QD administration, the upper limit value of the dose per administration is preferably 275 mg, more preferably 250 mg, still more preferably 225 mg, even still more preferably 200 mg, and even still further more preferably 150 mg.
(A3) The present invention provides a method for using Compound A for the treatment of acute myeloid leukemia, the method including administrating Compound A to a subject (a mammal including a human) requiring the treatment of acute myeloid leukemia one time a day before having a meal at a dose per administration of 50 to 300 mg.
(B3) The present invention provides a method for treating acute myeloid leukemia, including administering Compound A to a subject (a mammal including a human) requiring the treatment of acute myeloid leukemia one time a day before having a meal at a dose per administration of 50 to 300 mg.
(C3) The present invention provides the use of Compound A for producing an antitumor agent for acute myeloid leukemia, in which in the administration one time a day before having a meal, the dose per administration is 50 to 300 mg.
(D3) The present invention provides Compound A for using in the curing of acute myeloid leukemia, in which in the administration one time a day before having a meal, the dose per administration is 50 to 300 mg.

Regarding the administration method, the above-described dose per day can be administered daily for 28 days as one cycle.

Examples of the formulation form of the antitumor agent for acute myeloid leukemia according to the embodiment of the present invention include an oral agent, and examples thereof include a capsule agent. The administration formulation form can be manufactured by a conventional formulation manufacturing method known to those skilled in the art.

The antitumor agent for acute myeloid leukemia according to the embodiment of the present invention can be effectively used for the treatment of acute myeloid leukemia. The antitumor agent for acute myeloid leukemia according to the embodiment of the present invention can be used as anticancer agent.

The present invention provides a method for administering an antitumor agent to a patient with acute myeloid leukemia, in which the antitumor agent contains Compound A and a dose per administration of 25 to 225 mg is administered two times a day or a dose per thrice-daily administration of 20 to 150 mg is administered three times a day.

The present invention provides a method for using Compound A for the treatment of acute myeloid leukemia, the method including administering Compound A to a subject (a mammal including a human) requiring the treatment of acute myeloid leukemia two times a day at a dose per administration of 25 to 225 mg or three times a day at a dose per administration of 20 to 150 mg.

The present invention provides a method for treating acute myeloid leukemia, including administering Compound A to a subject (a mammal including a human) requiring the treatment of acute myeloid leukemia two times a day at a dose per administration of 25 to 225 mg or three times a day at a dose per administration of 20 to 150 mg.

The present invention provides the use of Compound A for the production of an antitumor agent for acute myeloid leukemia, in which in the twice-daily administration, the dose per administration is 25 to 225 mg or in the thrice-daily administration, the dose per administration is 20 to 150 mg.

The present invention provides Compound A for using in the curing of acute myeloid leukemia, in which in the twice-daily administration, the dose per administration is 25 to 225 mg or in the thrice-daily administration, the dose per administration is 20 to 150 mg. Examples

The present invention will be described in more detail below with reference to Examples; however, the present invention is not limited to these Examples.

### <Preparation of succinate salt of Compound A1>

(S,E)-N-(1-((5-(2-((4-cyanophenyl)amino)-4-(propylamino)pyrimidin-5-yl)-4-pentyn-1-yl)amino)-1-oxopropan-2-yl)-4-(dimethylamino)-N-methyl-2-butenamide was synthesized according to the method disclosed in Examples of Patent Document 4, converted to a succinate salt thereof according to the method described in Examples in Patent Document 2, and used in the following tests.

### <Preparation of oral agent>

The succinate salt of Compound A was used as a capsule formulation to obtain an oral agent.

This oral agent was used in the following curing. The curing has been carried out in United States of America at John's Hopkins University (JH) in Baltimore, Maryland, Pennsylvania University (UPENN) in Philadelphia, Pennsylvania, Northwestern University (NW) in Chicago, Illinois, and the University of California, San Francisco (UCSF) in San Francisco, California.

### <Reference test 1>

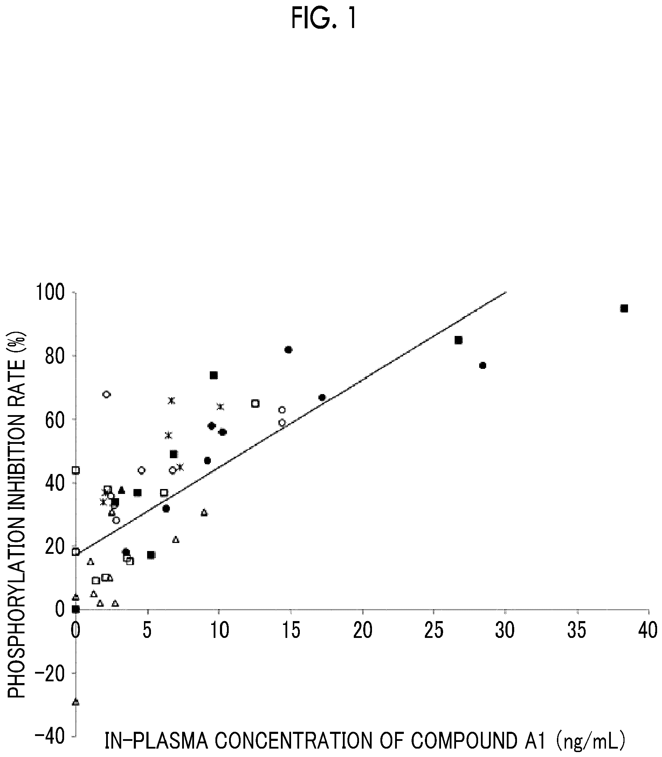

For 7 patients (patients 1 to 7) who were repeatedly administered with 10 mg of Compound A1 one time a day or 20 mg one time a day in a clinical test involving patients with acute myeloid leukemia, in-plasma concentrations of Compound A1 were measured before administration and after 4 hours after the administration on day 1, day 8, day 15, and day 22 of the first cycle (one cycle is 28 days) and on day 1 of the second cycle, and on the end day of the administration of Compound A1. In addition, a plasma inhibitory activity (PIA) test (Blood, 2006, Vol. 108, p3477 to 3483) was carried out using plasma samples from the same patients at the same time points. A graph showing a correlation between the in-plasma concentrations and the PIA test results is shown Fig. 1. In Fig. 1, open circles, open rectangles, open triangles, filled circles, filled triangles, filled rectangles, and cross symbols indicate the results of patients 1 to 7, respectively. No serious side effects were observed in the treated patients.

As shown in the PIA test results of Fig. 1, it can be seen that the phosphorylation inhibition rate reaches 100% in a case where the in-plasma concentration exceeds 30 ng/mL based on the regression line and thus in order to exhibit a remarkable FLT3 phosphorylation inhibition rate, an in-plasma concentration of 40 ng/mL or more is required.

### <Example 1>

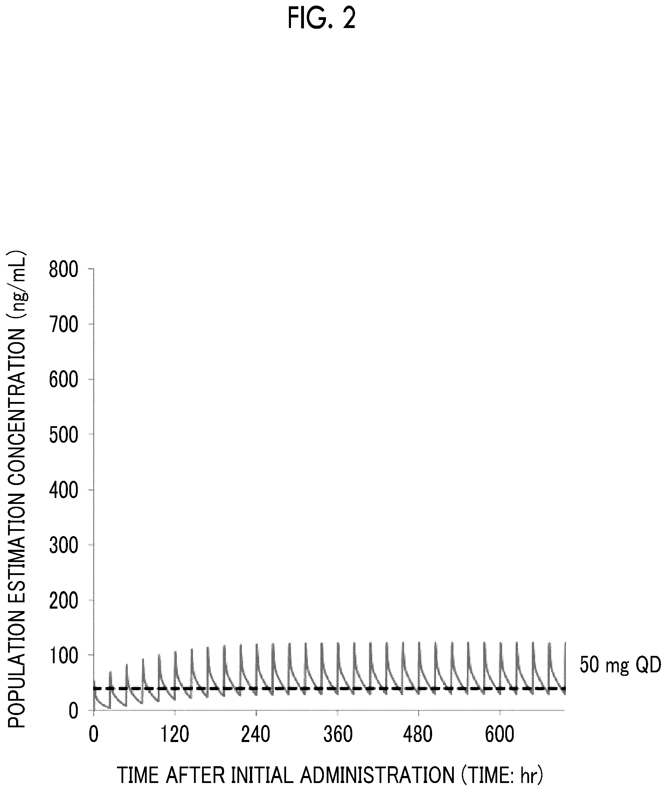

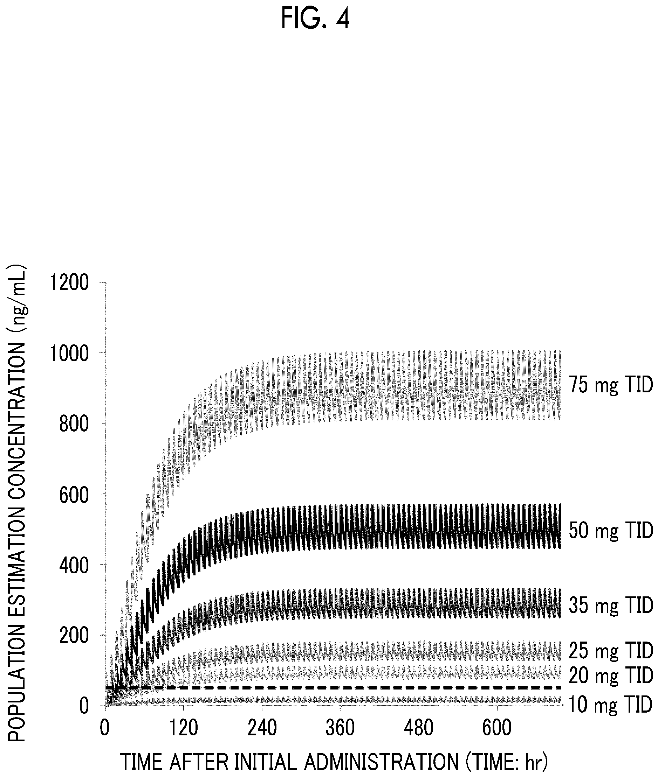

Population pharmacokinetic analysis was carried out using 198 pieces of in-plasma concentration data of Compound A1 obtained from 10 patients with acute myeloid leukemia. The population pharmacokinetic analysis software used was NONMEM (registered trade mark) (ICON Development Solutions Co., Ltd., Software version 7.3). Simulations were carried out using parameters estimated by population pharmacokinetic analysis, in order to study the doses at which the in-plasma drug concentration exceeding 40 ng/mL, which is the target value at which drug efficacy is expected, is obtained. Figs. 2 to 4 show the results of the simulation in which the change in drug concentration was simulated at the time of the QD administration of 50 mg per administration one time a day (QD), at the time of the BID administration of 25 to 75 mg per administration two times a day, and at the time of the TID administration of 10 to 75 mg three times a day. In Figs. 2 to 4, the dotted line indicates a line of 40 ng/mL.

As a result of the simulation, it was predicted that in terms of the population average value, the dose exceeding 40 ng/mL was 25 mg or more per administration at the time of the BID administration and 20 mg or more per administration at the time of the TID administration. On the other hand, it was suggested that at the time of the QD administration, a state of less than 40 ng/mL occurred at 50 mg per administration. As a result, it has been found that the divided administration is effective as a method for maintaining the in-plasma drug concentration exceeding 40 ng/mL while reducing the dose per day as much as possible.

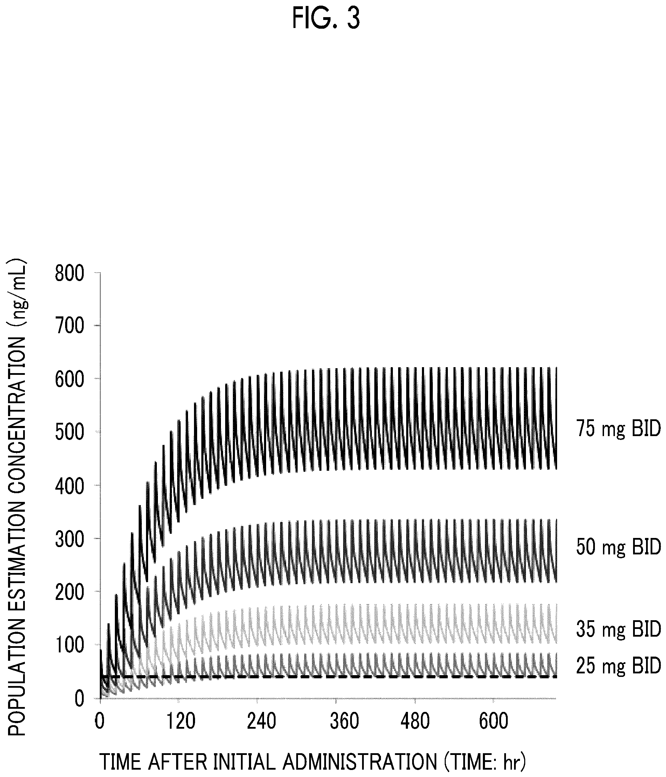

In the BID administration of Fig. 3, it can be estimated that the target in-plasma drug concentration can be obtained even at a low dose, the fluctuation range of the in-plasma concentration of Compound A1 is narrow, a sufficient curing effect can be expected, and unfavorable side effects can be suppressed.

### <Example 2: BID administration test 1>

A patient with acute myeloid leukemia is subjected to the BID administration of Compound A of 25 to 225 mg per administration before having a meal two times a day. A preferred effect (for example, a decrease in the proportion of blast cells in the bone marrow, or a curing effect equal to or higher than PR) is confirmed. The specific doses per administration are 50 mg, 75 mg, 100 mg, or 150 mg.

Patients have received chemotherapy with cytarabine, daunorubicin, idarubicin, and the like as preliminary curing, and some patients have not reached CR, CRi, CRp, or PR by preliminary curing.

### <Determination of administration and curing effect>

The curing effect is determined by the following criteria.

The bone marrow puncture sample is evaluated and determined according to the following criteria.

Complete Response (CR): a state in which Auer bodies are not observed in 5% or less of myeloblasts, and the neutrophil count and the platelet count are respectively 1,000 counts/µL or more and 100,000 counts/µL or more.

Complete Response with incomplete platelet recovery (CRpy): a state in which myeloblasts are 5% or less and the neutrophil count is 1,000 counts/µL or more, but the platelet count is 100,000 counts/µL or less.

Complete Response with incomplete hematological recovery (CRi): a state in which myeloblasts are 5% or less and there is no blood transfusion dependency, but the neutrophil count is 1,000 counts/µL or less.

Partial Response (PR): a state in which myeloblasts have been reduced by 50% or more to be 5% to 25% in the bone marrow puncture sample.

### <Result>

One 75-year old male patient with acute myeloid leukemia was subjected to the BID administration of Compound A1 of 75 mg per administration before having a meal two times a day. The administration period was 35 days. When a bone marrow examination was carried out on day 29 of the administration, the proportion of blast cells in the bone marrow decreased from 55% before administration to 18%. This patient was a patient who received a remission induction therapy with which cytarabine and idarubicin were administered and a remission induction therapy with which azacytidine was administered, before the administration of Compound A1, but was not completely cured, and this result suggests the efficacy of Compound A1.

### <Example 3: TID administration test 1>

A patient with acute myeloid leukemia is subjected to the TID administration of Compound A1 of 20 to 150 mg per administration before having a meal three times a day. A preferred effect (for example, a decrease in the proportion of blast cells in the bone marrow, or a curing effect equal to or higher than PR) is confirmed.

Patients have received chemotherapy with cytarabine, daunorubicin, idarubicin, and the like as preliminary curing, and some patients have not reached CR, CRi, or CRp by preliminary curing.

### <Example 3: QD administration test 1>

A patient with acute myeloid leukemia is subjected to the QD administration of Compound A1 of 50 to 300 mg per administration before having a meal one time a day. The dose is more specifically 50 mg, 75 mg, 100 mg, 150 mg, 225 mg, or 300 mg. A preferred effect (for example, a decrease in the proportion of blast cells in the bone marrow, or a curing effect equal to or higher than PR) is confirmed.

Patients have received chemotherapy with cytarabine, daunorubicin, idarubicin, and the like as preliminary curing, and some patients have not reached CR, CRi, or CRp by preliminary curing.

### [New pharmaceutical use of compound of General Formula [1] and salt thereof]

The compound of General Formula [1] and a salt thereof are useful for the treatment of an FLT3 mutation-positive cancer (WO 2016/027904 is referenced, which is incorporated in the present specification) and for the treatment of the mutation-positive cancer resistant to the existing drugs.

The present invention also provides a treatment agent and an anticancer agent for a mutation-positive cancer resistant to the existing drugs, which include the compound of General Formula [1] and a salt thereof, and a method for treating an FLT3 mutation-positive cancer in a subject, the method including administering the anticancer agent to a subject (preferably a human).

The present invention also provides a compound of General Formula [1] and a salt thereof for use in the method for treating the mutation-positive cancer resistant to existing drugs described above. As the compound of General Formula [1] and a salt thereof, those described above are used, and the same applies to suitable compounds thereof.

Examples of the mutation-positive cancer include hematological cancer, and examples of the hematological cancer include acute lymphocytic leukemia (ALL), acute myeloid leukemia (AML), acute promyelocytic leukemia (APL), chronic lymphocytic leukemia (CLL), chronic myeloid leukemia (CML), chronic neutrophilic leukemia (CNL), acute undifferentiated leukemia (AUL), anaplastic large cell lymphoma (ALCL), prolymphocytic leukemia (PML), juvenile myelomonocytic leukemia (JMML), adult T-cell ALL, myelodysplastic syndrome (MDS), and myeloproliferative disorder (MPD). Among the above, AML is preferable.

Here, examples of the existing drug include Gilteritinib, Quizartinib, and Midostaurin. Gilteritinib is 6-ethyl-3-[3-methoxy-4-[4-(4-methylpiperazin-1-yl)piperidin-1-yl]anilino]-5-(oxan-4-ylamino )pyrazine-2-carboxamide. Quizartinib is 1-(5-(tert-butyl)isoxazol-3-yl)-3-(4-(7-(2-morpholinoethoxy)benzo[d]imidazo[2,1-b]thiazol-2-yl)phenyl)urea. Midostaurin is 4'-N-benzoyl staurosporine.

In a case where an anticancer drug is administered to a patient, a mutation-positive cancer may occur, which diminishes the effect (the inhibitory action) of the anticancer drug. The mutation-positive cancer in which the inhibitory effect of Gilteritinib, Quizartinib, and Midostaurin is diminished include a cancer carrying a mutation of FLT3-ITD + D698N or FLT3-ITD + N676T. FLT3-ITD + D698N has, in addition to the FLT3-ITD mutation, a mutation in which the aspartic acid residue at the position corresponding to the 698th position of wild-type FLT3 is substituted with an asparagine residue, and FLT3-ITD + N676T has, in addition to the FLT3-ITD mutation, a mutation in which the asparagine residue at the position corresponding to the 676th position of wild-type FLT3 is substituted with a threonine residue.

### [Example: evaluation of inhibitory effect of FLT3 inhibitor on proliferation of mutant FLT3-expressing 32D cell]

### <Test substance>

As the test substance, a succinate salt of Compound A1, Quizartinib (manufactured by ChemieTek, LLC), Gilteritinib (manufactured by ChemieTek, LLC), and Midostaurin (manufactured by LKT Laboratories, Inc.) were used.

### <Experimental method>

32D cell line expressing FLT3 gene mutations (FLT3-ITD + D698N, + N676T) found according to the random mutagenesis analysis method was cultured for a predetermined period of time (setting: 37°C and 5% CO₂, steam saturated), and then the cells were plated on a 384-well plate at a cell concentration of 299 cells/well. The random mutagenesis analysis method was carried out according to the following reference document.

### (Reference document)

Smith CC, Wang Q, Chin CS, et al., Validation of ITD mutations in FLT3 as a therapeutic target in human acute myeloid leukemia. Nature. 2012; 485(7397): 260 to 263.

Each of the test substances was dissolved in dimethyl sulfoxide (DMSO) to prepare a DMSO solution containing 20 mmol/L of the test substance. After serially diluting with DMSO and further diluting with a medium containing 10% serum, each of the test substance solutions having a concentration 10 times the final treatment concentration was prepared. As the medium, RPMI 1640 medium (manufactured by Thermo Fisher Scientific, Inc., product number: 11875-093) was used. Each of the test substance solutions was added to each well so that the concentration was in a dilution series with the common ratio of 1/2. In addition, a group (a positive control group) in which only DMSO containing no test substance was added to a well in which cells were plated and a group (a negative control group) in which only DMSO containing no test substance was added to a well in which only the medium was added were prepared. On day 3 after adding the test substance solution, the intracellular ATP level was measured with "Cell" ATP Assay reagent Ver. 2 (manufactured by TOYO B-Net Co., Ltd.) to evaluate cell viability.

The suppression rate was determined by assuming that the amount of luminescence signal in the negative control group corresponded to the suppression of cell viability by 100% and the amount of luminescence signal in the positive control group corresponded to the suppression of cell viability by 0%. The concentration (IC50 value) at which cell viability is suppressed by 50% was calculated using XLFit (registered trade mark) software Ver. 3 (manufactured by ITOCHU Techno-Solutions Corporation).

From the obtained IC50 value (unit: nmol/L), the inhibitory effect of the FLT3 inhibitor on the proliferation of the mutant FLT3-expressing 32D cell was determined according to the effect determination criteria in Table 1. The obtained results are shown in Table 2.

**[Table 1]**

| IC50 value (nmol/L) | Effect determination |
|---|---|
| 0.1 or more and less than 0.5 | ++++ |
| | (Strong) |
| 0.5 or more and less than 1.0 | +++ |
| 1.0 or more and less than 5.0 | ++ |
| 5.0 or more and less than 10 | + |
| 10 or more and less than 50 | - |
| 50 or more and less than 100 | -- |
| | (Weak) |

**[Table 2]**

| Mutation type | Compound A1 | Gilteritinib | Quizartinib | Midostaurin |
|---|---|---|---|---|
| FLT3-ITD | ++++ | ++ | +++ | + |
| FLT3-ITD + D698N | +++ | - | +++ | - |
| FLT3-ITD + N676T | +++ | + | + | - |

### <Results and discussion>

Compound A1 exhibits a strong inhibitory effect on the proliferation of the FLT3-ITD + D698N-expressing 32D cell in which the cell growth inhibitory effect of Gilteritinib or Midostaurin is diminished as compared with the FLT3-ITD-expressing 32D cell. In addition, compound A1 also exhibits a strong inhibitory effect on the proliferation of the FLT3-ITD + N676T-expressing 32D cell in which the cell growth inhibitory effect of Quizartinib or Midostaurin is significantly diminished.

As described above, it has been suggested that Compound A1 is effective for the mutation-positive cancer having FLT3-ITD + D698N, which is the mutation resistant to Gilteritinib and Midostaurin, or FLT3-ITD + N676T, which is the mutation resistant to Quizartinib and Midostaurin.

The antitumor agent according to the embodiment of the present invention is useful because it has a curing effect on acute myeloid leukemia.

## Claims

1. An antitumor agent for acute myeloid leukemia, comprising a compound represented by General Formula [1] or a salt thereof,
wherein in a twice-daily administration, a dose per administration is 25 to 225 mg, or
in a thrice-daily administration, the dose per administration is 20 to 150 mg, 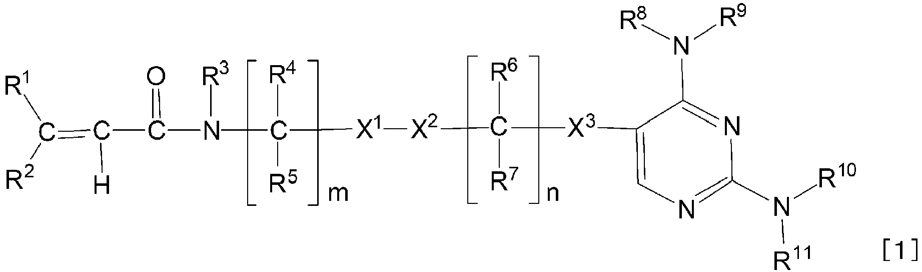 in the formula,
R¹ represents a hydrogen atom or a C₁₋₆ alkyl group which may be substituted,
R² represents a hydrogen atom, a C₁₋₆ alkyl group which may be substituted, a C₂₋₆ alkenyl group which may be substituted, or a C₂₋₆ alkynyl group which may be substituted,
R³ represents a hydrogen atom, a C₁₋₆ alkyl group which may be substituted, a C₂₋₆ alkenyl group which may be substituted, or a C₂₋₆ alkynyl group which may be substituted,
m represents an integer of 1 to 3,
m pieces of R⁴'s may be the same or different from each other and represent a hydrogen atom or a C₁₋₆ alkyl group which may be substituted, and one R⁴ selected from the m pieces of R⁴'s may be combined together with R³ to form a C₁₋₆ alkylene group which may be substituted,
m pieces of R⁵'s may be the same or different from each other and represent a hydrogen atom, a C₁₋₆ alkyl group which may be substituted, a C₂₋₆ alkenyl group which may be substituted, or a C₂₋₆ alkynyl group which may be substituted,
X¹ represents an oxygen atom, N(R²⁰) (in the formula, R²⁰ represents a hydrogen atom, a C₁₋₆ alkyl group which may be substituted, a C₂₋₆ alkenyl group which may be substituted, or a C₂₋₆ alkynyl group which may be substituted), C(=O), C(=O)-N(R²⁰) (in the formula, R²⁰ has the same meaning as the above), or a bond,
X² represents a C₁₋₆ alkylene group which may be substituted, a divalent alicyclic hydrocarbon group which may be substituted, or a divalent aromatic hydrocarbon group which may be substituted,
n represents an integer of 0 to 3,
n pieces of R⁶'s may be the same or different from each other and represent a hydrogen atom or a C₁₋₆ alkyl group which may be substituted,
n pieces of R⁷'s may be the same or different from each other and represent a hydrogen atom or a C₁₋₆ alkyl group which may be substituted,
X³ represents a C₁₋₆ alkylene group which may be substituted, a C₂₋₆ alkenylene group which may be substituted, a C₂₋₆ alkynylene group which may be substituted, or N(R²⁰)-C(=O) (in the formula, R²⁰ has the same meaning as the above),
R⁸ represents a hydrogen atom, a C₁₋₆ alkyl group which may be substituted, a C₂₋₆ alkenyl group which may be substituted, or a C₂₋₆ alkynyl group which may be substituted,
R⁹ represents a C₁₋₆ alkyl group which may be substituted, a C₂₋₆ alkenyl group which may be substituted, a C₂₋₆ alkynyl group which may be substituted, or a C₃₋₈ cycloalkyl group which may be substituted,
R⁸ and R⁹ may be combined together with a nitrogen atom to which R⁸ and R⁹ are bonded, to form a cyclic amino group which may be substituted,
R¹⁰ represents a hydrogen atom, a C₁₋₆ alkyl group which may be substituted, a C₂₋₆ alkenyl group which may be substituted, or a C₂₋₆ alkynyl group which may be substituted, and
R¹¹ represents a C₁₋₆ alkyl group which may be substituted, a C₂₋₆ alkenyl group which may be substituted, a C₂₋₆ alkynyl group which may be substituted, a C₃₋₈ cycloalkyl group which may be substituted, an aryl group which may be substituted, or a heterocyclic group which may be substituted.

2. The antitumor agent according to claim 1,
wherein in the twice-daily administration, the dose per administration is 35 to 150 mg.

3. The antitumor agent according to claim 1,
wherein in the twice-daily administration, the dose per administration is 35 to 100 mg.

4. The antitumor agent according to any one of claims 1 to 3,
wherein R¹⁰ is a hydrogen atom, and
X¹ is C(=O)-N(R²⁰) (in the formula, R²⁰ represents a hydrogen atom, a C₁₋₆ alkyl group which may be substituted, a C₂₋₆ alkenyl group which may be substituted, or a C₂₋₆ alkynyl group which may be substituted).

5. The antitumor agent according to any one of claims 1 to 4,
wherein X³ is a C₂₋₆ alkynylene group which may be substituted.

6. The antitumor agent according to any one of claims 1 to 5,
wherein the compound represented by General Formula [1] is (S,E)-N-(1-((5-(2-((4-cyanophenyl)amino)-4-(propylamino)pyrimidin-5-yl)-4-pentyn-1-yl)ami no)-1-oxopropan-2-yl)-4-(dimethylamino)-N-methyl-2-butenamide.

7. The antitumor agent according to claim 1,
wherein in the thrice-daily administration, the dose per administration is 35 to 150 mg.

8. The antitumor agent according to claim 1,
wherein in the thrice-daily administration, the dose per administration is 35 to 100 mg.

9. The antitumor agent according to any one of claims 1 to 8,
wherein the antitumor agent is an oral agent.
